# EUROPEAN PATENT APPLICATION

(11) **EP 4 691 421 A1**
(43) Date of publication of application: **11.02.2026**
(21) Application number: 24810052.1
(22) Date of filing: 02.04.2024
(51) Int. Cl.: A61F 2/01, A61B 17/22

(54) **ANTI-EMBOLISM FILTER AND FILTERING MEMBRANE**

(30) Priority: 19.05.2023 CN 202310572258
(71) Applicant: Mitrassist Lifesciences Limited, Shanghai 201807 (CN)
(72) Inventor: WANG, Zehui, Shanghai 201807 (CN); SHEN, Bin, Shanghai 201807 (CN); ZHAO, Xiangqian, Shanghai 201807 (CN)
(74) Representative: Gualeni, Nadia
(86) International application number: PCT/CN2024/085561
(87) International publication number: WO 2024/239804

(57) **Abstract**

The present disclosure provides an anti-embolism filter and a filtering membrane. The anti-embolism filter comprises a stent and a filtering membrane. The filtering membrane comprises an outer membrane and an inner membrane. The outer membrane is connected to the stent, the inner membrane is connected to the outer membrane, and an accommodating space is formed between the inner membrane and the outer membrane. According to the present disclosure, the filtering membrane blocks embolism particles from entering the brain via blood flow, and by means of the inner membrane and the outer membrane, the embolism particles are wrapped in the accommodating space formed by the inner membrane and the outer membrane and are thus collected and completely taken out of the body.

## Description

### CROSS-REFERENCE TO RELATED APPLICATION

The present disclosure claims priority to Chinese patent Application No. 2023105722588, filed with the Chinese Patent Office on May 19, 2023, entitled "ANTI-EMBOLISM FILTER AND FILTERING MEMBRANE", the entire contents of which are incorporated herein by reference.

### TECHNICAL FIELD

The present disclosure relates to the technical field of medical devices, and specifically to an anti-embolism filter and a filter membrane.

### BACKGROUND ART

During cardiac interventional surgery, embolic particles such as thrombi, calcifications, and atheromatous plaques may be expelled through surgical or catheter operations and enter the bloodstream, thereby causing embolism in the brain or other vital downstream organs through cerebral blood vessels. Cerebral embolism may lead to neuropsychological defects, stroke, or even death. Therefore, it is necessary to block embolic particles from entering the brain via the bloodstream during the surgery.

The existing embolic protection filters have the capability to block embolic particles, but the recovery effect of the embolic particles is not satisfactory.

### SUMMARY

The present disclosure provides an anti-embolism filter, including:
a stent; and
a filter membrane, wherein the filter membrane includes an outer membrane and an inner membrane; the outer membrane is connected to the stent; and the inner membrane is connected to the outer membrane, and an accommodation space is formed between the inner membrane and the outer membrane.

Optionally, a downstream end of the inner membrane in the blood flow direction is connected to the outer membrane.

An upstream end of the inner membrane in the blood flow direction is connected to the outer membrane or the stent by a connection component.

Optionally, a cavity is formed in the stent, wherein a downstream end of the inner membrane is connected to the outer membrane in the downstream end of the blood flow direction; a connection component is arranged between an upstream end of the inner membrane and an upstream end of either the outer membrane or the stent in the blood flow direction; and an accommodation space is formed between the inner membrane and the outer membrane in the cavity of the stent.

Optionally, a small opening and a large opening are formed in the inner membrane, wherein the small opening is located at the upstream end of the inner membrane in the blood flow direction; the small opening is connected to the connection component; and the large opening is located at the downstream end of the inner membrane in the blood flow direction and is connected to the outer membrane.

Optionally, the small opening of the inner membrane is located at a middle position of the outer membrane in a length direction; or
the small opening of the inner membrane is located near the middle position of the outer membrane in the length direction.

Optionally, the inner membrane includes a conical segment and a cylindrical segment which are connected to each other, the conical segment is located downstream of the cylindrical segment in the blood flow direction, the small opening is arranged on the cylindrical segment, and the large opening is arranged on the conical segment.

Optionally, the anti-embolism filter is configured to be switchable between a radially contracted state and a radially expanded state, and the cylindrical segment is configured to at least partially fit to an outer surface of the catheter during use.

Optionally, the conical segment is coaxial with the cylindrical segment.

Optionally, the connection component includes a connecting strap and/or a support rod.

Optionally, the connection component includes a connection wire; the connection wire includes a first connection segment; and the first connection segment is embedded in the inner membrane or attached to a surface of the inner membrane.

Optionally, the connection wire includes a second connection segment connected to the first connection segment, and the second connection segment is embedded in the outer membrane or attached to the surface of the inner membrane.

Optionally, the connection wire includes a third connection segment, with two ends connected to the first connection segment and the second connection segment respectively, and the third connection segment is located between the small opening of the inner membrane and an upstream end of the outer membrane in the blood flow direction.

Optionally, a plurality of connection components are provided, and the plurality of connection components are distributed in a circumferential direction along the inner membrane.

Optionally, the cavity is formed in the stent, and the filter membrane is located in the cavity, wherein the outer membrane is adhered to an inner surface of the stent.

Optionally, the anti-embolism filter is configured to be switchable between the radially contracted state and the radially expanded state.

Optionally, in an implanted state, the stent is in the radially expanded state, wherein an outer surface of the stent is configured to be in contact with an inner wall of vascular system of a patient; the outer membrane is configured to be circumferentially adherent to the inner surface of the stent; and the accommodation space is formed between the outer membrane and the inner membrane, and the accommodation space is located in the inner cavity.

The present disclosure provides a filter membrane for the anti-embolism filter, and the filter membrane is the foregoing filter membrane.

Optionally, the filter membrane is the filter membrane used in the foregoing anti-embolism filter.

Optionally, the filter membrane includes the outer membrane and the inner membrane; the outer membrane is adhered to an inner surface of the stent; and the accommodation space is formed between the outer membrane and the inner membrane.

Optionally, the outer membrane and the inner membrane are the same or different biocompatible fabrics.

The present disclosure provides an anti-embolism filter, including:
a stent capable of forming a cavity; and
a filter membrane arranged in the cavity of the stent, wherein the filter membrane includes an outer membrane and an inner membrane; the outer membrane is adhered to an inner surface of the stent; an accommodation space is formed between the outer membrane and the inner membrane; the inner membrane includes a conical segment and a cylindrical segment which are connected to each other; the conical segment is located downstream of the cylindrical segment in a blood flow direction; a downstream end of the conical segment in a blood flow direction is connected to a downstream end of the outer membrane in the blood flow direction; and a connection component is connected between an upstream end of the cylindrical segment in the blood flow direction and an upstream end of the outer membrane in the blood flow direction.

The present disclosure provides an anti-embolism filter assembly, and the anti-embolism filter assembly includes:
a delivery device;
a recovery device; and
the anti-embolism filter as described above, wherein
the anti-embolism filter is configured to be delivered in a delivery state to a desired position by the delivery device and change to an implanted state; and/or
the anti-embolism filter is configured to change from the implanted state to the delivery state by the recovery device.

### BRIEF DESCRIPTION OF DRAWINGS

In order to more clearly illustrate the technical solutions in the embodiments of the present disclosure, the drawings to be used in the embodiments of the present disclosure will be briefly introduced below. It should be understood that the following drawings only show certain embodiments of the present disclosure, and therefore should not be regarded as a limitation of the scope. For a person of ordinary skill in the art, other relevant drawings can be obtained according to these drawings without inventive efforts.
FIG. 1 is a structure schematic diagram of an anti-embolism filter in an implanted state provided by an embodiment of the present disclosure;
FIG. 2 is a structure schematic diagram of an anti-embolism filter in a delivery state provided by an embodiment of the present disclosure;
FIG. 3 shows a sectional view of a filter membrane of an anti-embolism filter provided by an embodiment of the present disclosure;
FIG. 4 shows a sectional view of an anti-embolism filter provided by another embodiment of the present disclosure;
FIG. 5 shows a sectional view of a filter membrane of an anti-embolism filter provided by another embodiment of the present disclosure; and
FIG. 6 shows a structure schematic diagram of a connection wire provided by an embodiment of the present disclosure.

### Reference numbers:

10- stent; 11- cavity; 20- filter membrane; 21- outer membrane; 22- inner membrane; 23-accommodation space; 211- downstream end of the outer membrane; 212- upstream end of the outer membrane; 221- downstream end of the inner membrane; 222- upstream end of the inner membrane; 223- small opening; 224- large opening; 225- conical segment; 226- cylindrical segment; 30- connection component; 31- first connection segment; 32- second connection segment; 33- third connection segment.

### DETAILED DESCRIPTION OF EMBODIMENTS

The technical solutions in the embodiments of the present disclosure will be described below in conjunction with the drawings in the embodiments of the present disclosure.

It should be noted that similar symbols and letters denote similar items in the following drawings, so that once an item is defined in a drawing, no further definition or explanation is required in the subsequent drawings. Meanwhile, in the description of the present disclosure, the terms "first" and "second", etc., are used only to distinguish descriptions, and are not to be understood as indicating or implying a relative importance.

The present disclosure provides an anti-embolism filter that both blocks embolic particles from entering the brain via the bloodstream and recovers embolic particles.

In some embodiments, the present disclosure provides an anti-embolism filter, including: a stent and a filter membrane, wherein the filter membrane includes an outer membrane and an inner membrane; the outer membrane is connected to the stent; and the inner membrane is connected to the outer membrane, and an accommodation space is formed between the inner membrane and the outer membrane.

In the present disclosure, the filter membrane blocks the embolic particles to prevent the embolic particles from entering the brain via the bloodstream; and an accommodation space is formed between the inner membrane and the outer membrane to warp the embolic particles for recovery and removal from the body. The small embolic particles can also be completely removed. It provides more thorough recovery and reduces the risk of the embolic particles entering the brain.

In some embodiments, the stent can include a shape memory alloy or a shape memory polymer.

In some embodiments, the filter membrane can be a porous polymer membrane, e.g., the porous polymer membrane can be a biocompatible fabric with a pore size between 50 microns and 300 microns; the porous polymer membrane can be a biocompatible fabric with a pore size between 100 microns and 200 microns; or the porous polymer membrane can be a polymer filter membrane.

In some embodiments, the inner membrane and the outer membrane can be the same, e.g., both of which can be biocompatible fabrics with the pore sizes between 50 microns and 300 microns; or both of which can be biocompatible fabrics with the pore size between 100 microns and 200 microns.

In some embodiments, the inner membrane and the outer membrane can be different, e.g., the inner membrane can be the biocompatible fabric with the pore size between 50 microns and 300 microns, and the outer membrane can be a non-porous membrane.

In some embodiments, a downstream end of the inner membrane in the blood flow direction is connected to the outer membrane, and the upstream end of the inner membrane in the blood flow direction is connected to the outer membrane or the stent by a connection component.

In the present disclosure, the connection component can prevent the inner membrane from shifting under the impact of blood flow, thereby maintaining the inner membrane in the outer membrane, which in turn allows an accommodation space for accommodating embolic particles to always be formed between the inner membrane and the outer membrane.

In some embodiments, a small opening and a large opening are formed in the inner membrane, wherein the small opening is located at the upstream end of the inner membrane in the blood flow direction; the small opening is connected to the connection component; and the large opening is located at the downstream end of the inner membrane in the blood flow direction and is connected to the outer membrane.

In some embodiments, a cavity is formed in the stent, wherein a downstream end of the inner membrane is connected to the outer membrane in the downstream end of the blood flow direction; a connection component is arranged between an upstream end of the inner membrane and an upstream end of either the outer membrane or the stent in the blood flow direction; and an accommodation space is formed between the inner membrane and the outer membrane in the cavity of the stent.

In some embodiments, the small opening of the inner membrane is located at a middle position of the outer membrane in a length direction; or the small opening of the inner membrane is located near the middle position of the outer membrane in the length direction.

In the present disclosure, the downstream end of the inner membrane in the blood flow direction extends towards the upstream end, and the small opening faces the blood flow direction for accessing the catheter. When the catheter is accessed, the small opening closes under the force of blood flow impact, thereby preventing the passage of embolic particles in the bloodstream.

In some embodiments, the inner membrane includes a conical segment and a cylindrical segment connected to each other, wherein the conical segment is located downstream of the cylindrical segment in the blood flow direction; the small opening is arranged on the cylindrical segment; and the large opening is arranged on the conical segment.

In some embodiments, the anti-embolism filter is configured to be switchable between a radially contracted state and a radially expanded state, and the cylindrical segment is configured to at least partially fit to an outer surface of the catheter in use.

In the present disclosure, the cylindrical segment is a section of a flat structure, and is compressed and closed under the impact of blood flow when the catheter is not accessed, which prevents thrombus and other embolic particles in the bloodstream from flowing out through the small opening. When the catheter is accessed, the cylindrical segment has an overlapping segment with the catheter, which provides a better fit with the catheter, thereby preventing thrombus and other embolic particles from escaping from the gap between the catheter and the cylindrical segment. The diameter of the cylindrical segment can be adapted to the diameter of the accessing part of the catheter, which ensures that the catheter can pass through the cylindrical segment and also prevents the escape of the embolic particles.

In some embodiments, the conical segment is coaxial with the cylindrical segment. The conical segment and the cylindrical segment form a centrally symmetrical structure. There is no need to consider circumferential positioning when placing, and placing at any angle all allows the small opening to be located at the center, which is convenient for placement.

In some embodiments, the connection component includes a connecting strap and/or a support rod.

In some embodiments, the connection component includes a connection wire; the connection wire includes a first connection segment; and the first connection segment is embedded in the inner membrane or attached to a surface of the inner membrane.

In some embodiments, the connection wire includes a second connection segment connected to the first connection segment, and the second connection segment is embedded in the outer membrane or attached to the surface of the inner membrane.

In some embodiments, the connection wire includes a third connection segment, with two ends connected to the first connection segment and the second connection segment respectively, and the third connection segment is located between the small opening of the inner membrane and an upstream end of the outer membrane in the blood flow direction.

In some embodiments, a plurality of connection components are provided, and the plurality of connection components are distributed in a circumferential direction along the inner membrane.

In some embodiments, the cavity is formed in the stent, and the filter membrane is located in the cavity, wherein the outer membrane is adhered to an inner surface of the stent.

In some embodiments, the anti-embolism filter is configured to be switchable between the radially contracted state and the radially expanded state.

In some embodiments, in an implanted state, the stent is in the radially expanded state, wherein an outer surface of the stent is configured to be in contact with an inner wall of vascular system of a patient; the outer membrane is configured to be circumferentially adherent to the inner surface of the stent; and the accommodation space is formed between the outer membrane and the inner membrane, and the accommodation space is located in the inner cavity.

The present disclosure provides a filter membrane for the anti-embolism filter, and the filter membrane is the filter membrane as used in any one of the above embodiments.

In some embodiments, the filter membrane can be a porous polymer membrane, e.g., the porous polymer membrane can be a biocompatible fabric with a pore size between 50 microns and 300 microns or a biocompatible fabric with a pore size between 100 microns and 200 microns.

In some embodiments, the inner membrane and the outer membrane can be the same or different, e.g., the inner membrane and the outer membrane can both be biocompatible fabrics with the pore sizes between 50 microns and 300 microns (such as between 100 microns and 200 microns). Alternatively, the inner membrane can be a biocompatible fabric with the pore size between 50 microns and 300 microns (such as between 100 microns and 200 microns), and the outer membrane can be a non-porous membrane.

In the present disclosure, the filter membrane blocks the embolic particles to prevent the embolic particles from entering the brain via the bloodstream; and the embolic particles are wrapped in the accommodation space formed by the inner membrane and the outer membrane, so as to be recovered and removed out of the body.

The present disclosure provides an anti-embolism filter, including: a stent capable of forming a cavity; and a filter membrane, arranged in the cavity of the stent, wherein the filter membrane includes an outer membrane and an inner membrane; the outer membrane is adhered to an inner surface of the stent; an accommodation space is formed between the outer membrane and the inner membrane; the inner membrane includes a conical segment and a cylindrical segment connected to each other; the conical segment is located downstream of the cylindrical segment in a blood flow direction; a downstream end of the conical segment in a blood flow direction is connected to a downstream end of the outer membrane in the blood flow direction; and a connection component is connected between an upstream end of the cylindrical segment in the blood flow direction and an upstream end of the outer membrane in the blood flow direction.

The present disclosure provides an anti-embolism filter assembly, and the anti-embolism filter assembly includes:
a delivery device;
a recovery device; and
the anti-embolism filter as described above, wherein
the anti-embolism filter is configured to be delivered in a delivery state to a desired position by the delivery device and change to an implanted state; and/or
the anti-embolism filter is configured to change from the implanted state to the delivery state by the recovery device.

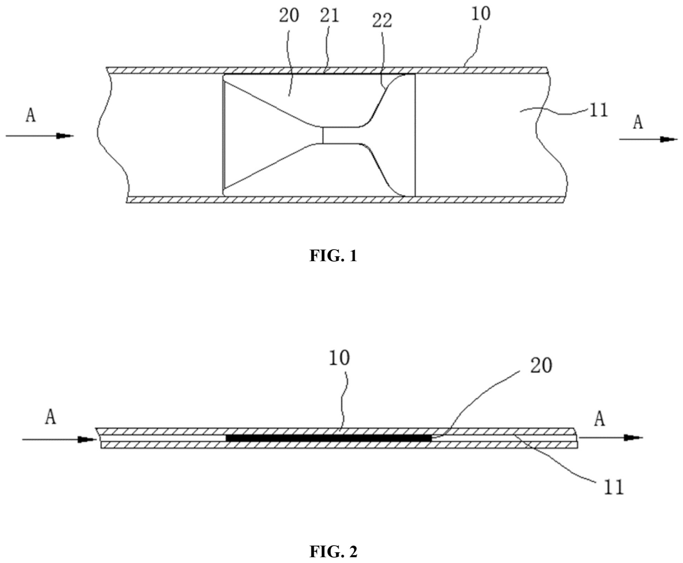

Referring to FIG. 1 and FIG. 2, FIG. 1 is a structure schematic diagram of an anti-embolism filter provided by the embodiment of the present disclosure, and FIG. 2 is a structure schematic diagram of an anti-embolism filter in a delivery state provided by the embodiment of the present disclosure.

The embodiment of the present disclosure provides an anti-embolism filter, including a stent 10 and a filter membrane 20, wherein the filter membrane includes an outer membrane 21 and an inner membrane 22, the outer membrane 21 is connected to the stent 10, the inner membrane 22 is connected to the outer membrane 21, and an accommodation space 23 is formed between the inner membrane 22 and the outer membrane 21.

The anti-embolism filter has a delivery state (as shown in FIG. 2) that can be radially contracted and an implanted state (as shown in FIG. 1) that can be radially expanded. When the anti-embolism filter is under the implanted state, the stent 10 and the filter membrane 20 expand radially; a radial support is formed between the stent 10 and the vascular wall; and the bloodstream is filtered after flowing through the filter membrane 20, so that embolic particles such as thrombus, calcified and atherosclerotic plaques during the surgery are blocked and accommodated in the accommodation space 23 formed between the inner membrane 22 and the outer membrane 21. After the surgery is completed, the anti-embolism filter changes from the implanted state to the delivery state through the corresponding recovery device. At this point, the stent 10 and the filter membrane 20 are contracted radially to wrap the embolic particles in the accommodation space 23 and eventually take them out of the body.

In other words, during a cardiac interventional procedure, as the anti-embolism filter expands radially, the embolic particles generated during the procedure can be captured by the filter, thereby preventing them from entering the bloodstream. By recovering the anti-embolism filter from the body of the patient, the filter changes to a radially contracting delivery state and retains the collected embolic particles in the accommodation space 23 to be taken out of the body.

In the anti-embolism filter provided by the embodiments of the present disclosure, the filter membrane 20 blocks the embolic particles to prevent the embolic particles from entering the brain via the bloodstream, and the embolic particles are wrapped in the accommodation space formed by the inner membrane 22 and the outer membrane 21, so as to be recovered and removed out of the body. Since the size of the filter pore of the filter membrane is smaller than that of the self-formed filter pores of stent in the prior art, the accommodation space formed by the filter membrane in the present disclosure can better capture and retain small embolic particles, and finer embolic particles can also be recovered and removed, which provides more thorough recovery, and reduces the risk of the embolic particles entering the brain.

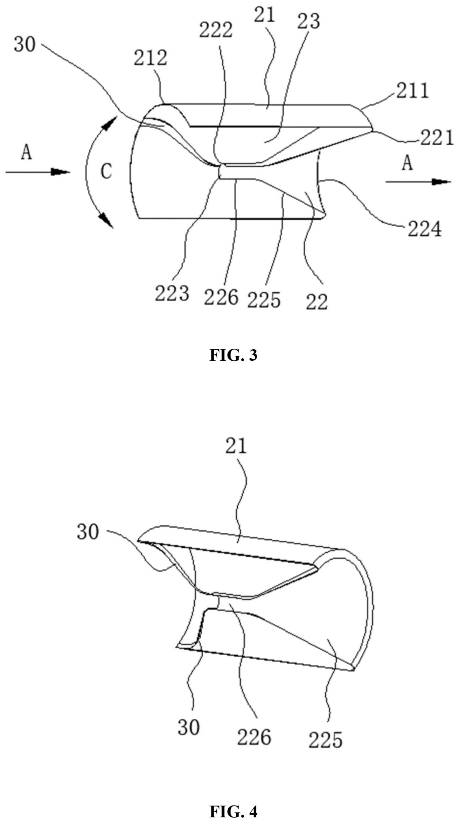

In some embodiments, referring to FIG. 3, a downstream end 221 of the inner membrane 22 in the blood flow direction A is connected to the outer membrane 21, and the inner membrane 22 is connected to the outer membrane 21 or the stent 10 by a connection component 30 at the upstream end 222 in the blood flow direction A. For ease of displaying the filter membrane structure, the stent is not shown in FIG. 3, and the outer membrane 21 is attached to the stent 10 in practical use.

The connection component 30 can prevent the inner membrane 22 from shifting under the impact of blood flow, maintaining the inner membrane 22 in the outer membrane 21, thereby allowing an accommodation space 23 for accommodating embolic particles to be continuously formed between the inner membrane 22 and the outer membrane 21.

In some examples, the connection component 30 includes a connecting strap and/or a support rod. The connection component 30 can be a flexible connecting strap, or a rigid support rod. The flexible connecting strap can play a pulling and/or tugging effect on the inner membrane 22 under the impact of blood flow. The rigid support rod can support the inner membrane 22 under the impact of blood flow.

Optionally, referring to FIG. 4, a plurality of connection components 30 can be provided, and the plurality of connection components 30 are distributed in a circumferential direction C along the inner membrane 22. Under the impact of the blood flow, the inner membrane 22 undergoes pulling and/or dragging and/or supporting forces at multiple locations, so as to ensure that the inner membrane 22 always remains in the outer membrane 21.

In some embodiments, referring to FIG. 3, a small opening 223 and a large opening 224 are formed in the inner membrane 22, wherein the small opening 223 is located at the upstream end 222 of the inner membrane 22 in the blood flow direction; the small opening 223 is connected to the connection component 30; and the large opening 224 is located at the downstream end 221 of the inner membrane 22 in the blood flow direction and is connected to the outer membrane 22.

The inner membrane 22 extends from the downstream end 221 towards the upstream end 222 in the blood flow direction A, and the small opening 223 faces the blood flow direction A for accessing the catheter. When the catheter is not inserted, the small opening 223 closes under the force of blood flow impact, thereby preventing the passage of embolic particles in the bloodstream.

Exemplarily, the large opening 224 of the inner membrane 22 can be attached to the downstream end of the outer membrane 21 in the blood flow direction A by pasting or suturing, and the inner membrane 22 extends from the large opening 224 to the small opening 223 in a counter-blood flow direction (a direction opposite to the blood flow direction A). The diameter of the inner membrane 22 decreases from the large opening 224 to the small opening 223.

In some embodiments, the small opening 223 of the inner membrane 22 is located at a middle position of the outer membrane 21 in a length direction; or the small opening 223 of the inner membrane 22 is located near the middle position of the outer membrane 21 in the length direction.

The small opening 223 of the inner membrane 22 is located at or near the middle position of the outer membrane 21 in the length direction. On the one hand, it ensures that the inner membrane 22 is in the inner cavity of the outer membrane 21. On the other hand, it prevents the inlet space at the upstream end of the outer membrane 21 from being affected. The embolic particles follow the blood flow to smoothly enter the inner cavity of the outer membrane 21, where they are blocked by the inner membrane 22 and accommodated within the accommodation space formed between the inner membrane 22 and the outer membrane 21.

In some embodiments, referring to FIG. 3, the inner membrane 22 includes a conical segment 225 and a cylindrical segment 226 which are connected to each other, wherein the conical segment 225 is located downstream of the cylindrical segment 226 in the blood flow direction A; the small opening 223 is arranged on the cylindrical segment 226; and the large opening 224 is arranged on the conical segment 225.

The cylindrical segment 226 is a section of a flat structure, and is compressed and closed under the impact of blood flow when the catheter is not inserted, which prevents thrombus and other embolic particles in the bloodstream from flowing out through the small opening 223. When the catheter is inserted, the cylindrical segment 226 has an overlapping segment with the catheter, which provides a better fit with the catheter, thereby preventing thrombus and other embolic particles from escaping from the gap between the catheter and the cylindrical segment. The diameter of the cylindrical segment 226 can be adapted to the diameter of the accessing part of the catheter, which ensures that the catheter can pass through the cylindrical segment 226 and also prevents the escape of the embolic particles.

In some embodiments, the conical segment 225 is coaxial with the cylindrical segment 226. The conical segment 225 and the cylindrical segment 226 form a centrally symmetrical structure. There is no need to consider circumferential positioning when placing, and the small opening 223 can be located at the center regardless of the placement angle, which is convenient for placement.

In other embodiments, the conical segment 225 and cylindrical segment 226 can also be non-coaxial. The conical segment 225 and the cylindrical segment 226 can be eccentric structures, in which case the small opening 223 can be placed a on top, requiring positioning during placement. The placement facilitates the passage of subsequent surgical instruments through the small opening 223, and the eccentric construction is more beneficial for the passage of subsequent surgical instruments.

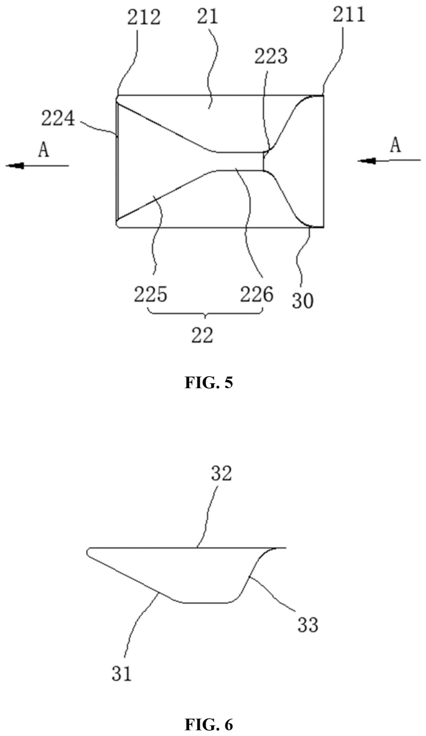

In some embodiments, referring to FIG. 5 and FIG. 6, the connection component 30 can include a connection wire, wherein the connection wire includes a first connection segment 31, and the first connection segment 31 is embedded in the inner membrane 22.

The connection wire further includes a second connection segment 32 connected to the first connection segment 31, and the second connection segment 32 is embedded in the outer membrane 21.

The connection wire further includes a third connection segment 33, with two ends connected to the first connection segment 31 and the second connection segment 32 respectively, and the third connection segment 33 is located between the small opening 223 of the inner membrane 22 and a downstream end 211 of the outer membrane 21 in the blood flow direction.

Optionally, the first connection segment 31, the second connection segment 32, and the third connection segment 33 can form a closure structure. The connection can be achieved by knotting, adhesion, or indirectly by connecting through a polymer filter membrane at the opening 223 of the cylindrical segment 226 to form the closure structure.

The connection wire can be directly fused and embedded in the polymer filter membrane, or be fixed to the surface of the filter membrane by adhesion or suturing. The connection wire is attached to the surface of the polymer membrane. It can be located on the outer side of the filter membrane 22 or on the inner side of the filter membrane 20, or it can be partly located on the outer side of the filter membrane 20 and partly on the inner side of the filter membrane 20. A preferred structures is that the first connection segment 31 and the second connection segment 32 are embedded at the middle of the filter membrane, with the polymer filter membrane wrapping the first connection segment 31 and the second connection segment 32.

The connection wire can adopt an elongated structure, and the elongated structure can be a single-strand or multi-strand twisted wire or cord with good flexibility, or a strip-shaped structure characterized by good pliability and the ability to bend and fold. Preferably, the maximum size of the cross section of the connection wire is between 0.05 and 0.2 mm. A plurality of elongated structures can be arranged circumferentially, in a number ranging from one to multiple. When multiple elongated structures are arranged, they can be distributed evenly or unevenly.

In some embodiments, the cavity 11 is formed in the stent 10, and the filter membrane 20 is located in the cavity 11, wherein the outer membrane 21 is adhered to an inner surface of the stent 10. In the expanded state of the stent 10, the outer membrane 21 can be cylindrical in shape.

The embodiment of the present disclosure further provides a filter membrane for the anti-embolism filter, and the filter membrane is the filter membrane 20 as described in the above embodiments.

The embodiment of the present disclosure further provides an anti-embolism filter, referring to FIG. 1- FIG. 6, including: a stent 10 capable of forming a cavity 11 under the expanded state, and a filter membrane 20 arranged in the cavity 11 of the stent 10, wherein the filter membrane 20 includes an outer membrane 21 and an inner membrane 22; the outer membrane 21 is adhered to an inner surface of the stent 10; an accommodation space 23 is formed between the outer membrane 21 and the inner membrane 20; the inner membrane 22 includes a conical segment 225 and a cylindrical segment 226 which are connected to each other; the conical segment 225 is located downstream of the cylindrical segment 226 in a blood flow direction A; a downstream end 221 of the conical segment 225 in a blood flow direction C is connected to a downstream end 211 of the outer membrane 21 in the blood flow direction; and a connection component is connected between an upstream end 223 of the cylindrical segment 226 in the blood flow direction A and an upstream end 212 of the outer membrane 21 in the blood flow direction A.

Filter pores can be formed in the inner membrane 22 and/or the outer membrane 21. The filter membrane 20 can be made of a polymer film. The inner membrane 22 can include a conical structure, and the closing end (small opening 223) of the conical structure is located at a middle position between the two ends of the outer membrane 21. The outer membrane can be cylindrical in shape, and the inner membrane is a conical structure. Both the outer membrane and the inner membrane are polymeric film structures. In order to maintain the conical structure of the inner membrane being invertedly inserted into the cylindrical structure of the outer membrane, a connection component 30 is arranged at an opening (small opening 223) of one end of the inner membrane, and the connection component 30 is connected to an opening (upstream end 212) of one end of the outer membrane. The length of the connection component 30 is set to ensure that the small opening of the inner membrane 22 is located within the outer membrane 21.

The anti-embolism filter can block embolic particles such as thrombus and dislodged plaques by the filter membrane, and capture these embolic particles and retain them in the accommodation space arranged between the inner membrane and the outer membrane, so as to remove the embolic particles such as the captured thrombus and dislodged plaques from the body during the recovery process, thereby ensuring that the distal blood vessels are protected throughout the entire surgical process. This maximally prevents the occurrence of distal embolism and achieves the protection of the distal arterial vessels throughout the body.

The foregoing is merely preferable embodiment of the present disclosure, and is not intended to limit the scope of the protection of the present disclosure. For those skilled in the art, the present disclosure may have various changes and variations. Any modifications, equivalent substitutions, improvements, etc., made within the spirit and principles of the present disclosure, shall be included in the scope of protection of the present disclosure. It should be noted that similar symbols and letters denote similar items in the following drawings, so that once an item is defined in a drawing, no further definition or explanation of it is required in the subsequent drawings.

The above are only the specific embodiments of the present disclosure, and the scope of protection of the present disclosure is not limited to this. Those skilled in the art can easily conceive of modifications or substitutions within the technical scope disclosed by the present disclosure, all of which shall fall within the protection scope of the present disclosure. Therefore, the protection scope of the present disclosure shall be determined by the scope of the claims.

It is to be noted that in the text, relationship terms such as first and second, etc., are used merely to distinguish one entity or operation from another, and do not necessarily require or imply the existence of any actual relationship or order between those entities or operations. Furthermore, the terms "include", "comprise" or any other variations thereof are intended to cover a nonexclusive inclusion, so that a process, method, object, or device including a series of elements includes not only those elements, but also other elements that are not explicitly listed, or the components that are inherent to this process, method, object, or device. Without further limitation, the component defined by a phrase "including a ..." does not exclude that the other same elements also exist in the process, method, object, or device including the component.

### Industrial Applicability

The present disclosure provides an anti-embolism filter and a filter membrane. The anti-embolism filter includes a stent and a filter membrane, wherein the filter membrane includes an outer membrane and an inner membrane, the outer membrane is connected to the stent, the inner membrane is connected to the outer membrane, and an accommodation space is formed between the inner membrane and the outer membrane. In the present disclosure, the filter membrane blocks the embolic particles to prevent the embolic particles from entering the brain via the bloodstream; and the embolic particles are wrapped in the accommodation space formed by the inner membrane and the outer membrane, thereby recovering all of them and removing them out of the body. This ensures the protection of the distal blood vessels throughout the entire surgical process, and maximally prevents the occurrence of distal embolism, thereby possessing excellent industrial applicability.

## Claims

1. An anti-embolism filter, comprising:
a stent; and
a filter membrane, wherein the filter membrane comprises an outer membrane and an inner membrane; the outer membrane is connected to the stent; the inner membrane is connected to the outer membrane; and an accommodation space is formed between the inner membrane and the outer membrane.

2. The anti-embolism filter according to claim 1, wherein
a downstream end of the inner membrane in a blood flow direction is connected to the outer membrane, and
an upstream end of the inner membrane in the blood flow direction is connected to the outer membrane or the stent by a connection component, wherein
optionally, a cavity is formed in the stent, a downstream end of the inner membrane is connected to the outer membrane in the downstream end of the blood flow direction; the connection component is arranged between the upstream end of the inner membrane and the upstream end of the outer membrane or the stent in the blood flow direction; and the accommodation space is formed between the inner membrane and the outer membrane in the cavity of the stent.

3. The anti-embolism filter according to claim 1 or 2, wherein
a small opening and a large opening are formed in the inner membrane, the small opening is located at the upstream end of the inner membrane in the blood flow direction; the small opening is connected to the connection component; and the large opening is located at the downstream end of the inner membrane in the blood flow direction and is connected to the outer membrane.

4. The anti-embolism filter according to claim 3, wherein
the small opening of the inner membrane is located at a middle position of the outer membrane in a length direction; or
the small opening of the inner membrane is located near the middle position of the outer membrane in the length direction.

5. The anti-embolism filter according to any one of claims 1-4, wherein
the inner membrane comprises a conical segment and a cylindrical segment which are connected to each other; the conical segment is located downstream of the cylindrical segment in the blood flow direction; the small opening is arranged on the cylindrical segment; and the large opening is arranged on the conical segment; and
optionally, the anti-embolism filter is configured to be switchable between a radially contracted state and a radially expanded state, and the cylindrical segment is configured to at least partially fit to an outer surface of a catheter during use.

6. The anti-embolism filter according to claim 5, wherein
the conical segment is coaxial with the cylindrical segment.

7. The anti-embolism filter according to any one of claims 2-6, wherein
the connection component comprises a connecting strap and/or a support rod.

8. The anti-embolism filter according to any one of claims 3-6, wherein
the connection component comprises a connection wire; the connection wire comprises a first connection segment; and the first connection segment is embedded in the inner membrane or attached to a surface of the inner membrane.

9. The anti-embolism filter according to claim 8, wherein
the connection wire comprises a second connection segment connected to the first connection segment, and the second connection segment is embedded in the outer membrane or attached to the surface of the inner membrane.

10. The anti-embolism filter according to claim 9, wherein
the connection wire comprises a third connection segment, with two ends connected to the first connection segment and the second connection segment respectively, and the third connection segment is located between the small opening of the inner membrane and the upstream end of the outer membrane in the blood flow direction.

11. The anti-embolism filter according to any one of claims 3-6, wherein
a plurality of connection components are provided, and the plurality of connection components are distributed in a circumferential direction along the inner membrane.

12. The anti-embolism filter according to any one of claims 1-6, wherein
the cavity is formed in the stent, the filter membrane is located in the cavity, and the outer membrane is adhered to an inner surface of the stent.

13. The anti-embolism filter according to any one of claims 1-12, wherein
the anti-embolism filter is configured to be switchable between the radially contracted state and the radially expanded state; and
optionally, in an implanted state, the stent is in the radially expanded state, wherein an outer surface of the stent is configured to be in contact with an inner wall of a vascular system of a patient; the outer membrane is configured to be circumferentially adherent to the inner surface of the stent; and the accommodation space is formed between the outer membrane and the inner membrane, and the accommodation space is located in the inner cavity.

14. A filter membrane for an anti-embolism filter, wherein the filter membrane is the filter membrane mentioned according to any one of claims 1-11;
optionally, the filter membrane is the filter membrane as used in the anti-embolism filter according to any one of claims 1-11;
optionally, the filter membrane comprises the outer membrane and the inner membrane; the outer membrane is adhered to an inner surface of the stent; and the accommodation space is formed between the outer membrane and the inner membrane; and
optionally, the outer membrane and the inner membrane are the same or different biocompatible fabrics.

15. An anti-embolism filter, comprising:
a stent capable of forming a cavity; and
a filter membrane arranged in the cavity of the stent, wherein the filter membrane comprises an outer membrane and an inner membrane; the outer membrane is adhered to an inner surface of the stent; an accommodation space is formed between the outer membrane and the inner membrane; the inner membrane comprises a conical segment and a cylindrical segment which are connected to each other; the conical segment is located downstream of the cylindrical segment in a blood flow direction; a downstream end of the conical segment in the blood flow direction is connected to a downstream end of the outer membrane in the blood flow direction; and a connection component is connected between an upstream end of the cylindrical segment in the blood flow direction and the upstream end of the outer membrane in the blood flow direction.

16. An anti-embolism filter assembly, wherein the anti-embolism filter assembly comprises:
a delivery device;
a recovery device; and
the anti-embolism filter according to any one of the claims 1-13 or claim 15, wherein
the anti-embolism filter is configured to be delivered in a delivery state to a desired position by the delivery device and change to an implanted state; and/or
the anti-embolism filter is configured to change from the implanted state to the delivery state by the recovery device.
